(19) Europäisches Patentamt
European Patent Office
Office européen des brevets

(11) EP 2 139 996 B1

(12) EUROPEAN PATENT SPECIFICATION

(45) Date of publication and mention of the grant of the patent:
**24.12.2014 Bulletin 2014/52**

(21) Application number: **08749160.1**

(22) Date of filing: **25.04.2008**

(51) Int Cl.:
***C12N 9/48*** $^{(2006.01)}$ ***C12N 9/58*** $^{(2006.01)}$
***A23J 3/00*** $^{(2006.01)}$ ***C12P 21/06*** $^{(2006.01)}$
***A23J 3/34*** $^{(2006.01)}$ ***A23L 1/03*** $^{(2006.01)}$

(86) International application number:
**PCT/EP2008/003386**

(87) International publication number:
**WO 2008/131938 (06.11.2008 Gazette 2008/45)**

(54) **PEPTIDASES FROM BASIDIOMYCETES**

PEPTIDASEN AUS BASIDIOMYCETEN

PEPTIDASES ISSUES DE BASIDIOMYCÈTES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **25.04.2007 EP 07008458**

(43) Date of publication of application:
**06.01.2010 Bulletin 2010/01**

(73) Proprietor: **Nestec S.A.**
**1800 Vevey (CH)**

(72) Inventors:
- **LINKE, Diana**
**31547 Bad Rehburg (DE)**
- **KRINGS, Ulrich**
**31628 Landesbergen (DE)**
- **ZORN, Holger**
**D-35392 Giessen (DE)**
- **RABE, Swen**
**78357 Muehlingen (DE)**
- **ULMER, Helge**
**91438 Bad Windsheim (DE)**

(74) Representative: **Mollet, Beat Max et al**
**Nestec S.A.**
**CT-IAM**
**Avenue Nestlé 55**
**1800 Vevey (CH)**

(56) References cited:


- **DATABASE CA [Online] CHEMICAL ABSTRACTS SERVICE, COLUMBUS, OHIO, US; NIZKOVSKAYA, O. P. ET AL: "Proteolytic activity of agaric fungi in a culture" XP002437885 retrieved from STN Database accession no. 81: 101864 & VYSSH. GRIBY IKH FIZIOL. AKTIV. SOEDIN. , 13-19. EDITOR(S): FEDOROV, AL. A. PUBLISHER: "NAUKA", LENINGRAD. OTD, LENINGRAD, USSR. CODEN: 28LLA6, 1973,**
- **ABE MAKOTO ET AL: "Proteases of Maitake (Grifola frondosa) responsible for breakdown of wheat flour dough and their reaction with gluten proteins." BIOSCIENCE, BIOTECHNOLOGY, AND BIOCHEMISTRY SEP 2003, vol. 67, no. 9, September 2003 (2003-09), pages 2018-2021, XP002437785 ISSN: 0916-8451**
- **ZORN HOLGER ET AL: "The secretome of Pleurotus sapidus" PROTEOMICS, vol. 5, no. 18, December 2005 (2005-12), pages 4832-4838, XP002437807 ISSN: 1615-9853**
- **NISHIWAKI TOSHIKAZU ET AL: "Debittering of enzymatic hydrolysates using an aminopeptidase from the edible basidiomycete Grifola frondosa." JOURNAL OF BIOSCIENCE AND BIOENGINEERING, VOL. 93, NO. 1, PP. 60-3. JOURNAL CODE: 100888800. ISSN: 1389-1723., 2002, XP002437786**
- **DATABASE CA [Online] CHEMICAL ABSTRACTS SERVICE, COLUMBUS, OHIO, US; YOSHIMOTO, TADASHI ET AL: "Proline iminopeptidase and its manufacture with mushrooms" XP002437886 retrieved from STN Database accession no. 115: 130673 & JP 03 108483 A (NAKANO VINEGAR CO., LTD., JAPAN) 8 May 1991 (1991-05-08)**


Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- **KAUSAR TASNIM ET AL: "Biomass production of Pleurotus sajor-caju by submerged culture fermentation" PAKISTAN JOURNAL OF SCIENTIFIC AND INDUSTRIAL RESEARCH, vol. 49, no. 2, March 2006 (2006-03), pages 116-119, XP009105706 ISSN: 0030-9885**

**SEQUENCE LISTING**

<110> Nestec S.A.
Linke, Diana
Krings, Ulrich
Zorn, Holger
Berger, R.G.
Rabe, Swen

Ulmer, Helge

<120> Peptidases from Basidiomycetes

<130> NO 8301/WO

<150> EP07008458.7
<151> 2007-04-25

<160> 6

<170> Patent In version 3.3

<210> 1
<211> 12
<212> PRT
<213> Grifola frondosa

<400> 1

```
Asp Gly Asn Ala Ser Pro Ala Ser Leu Ser Thr Lys
1               5                   10
```

<210> 2
<211> 15
<212> PRT
<213> Grifola frondosa

<400> 2

```
Gln Leu Ser Gly Leu Pro Ser Gly Thr Leu Asn Asp Leu Ala Arg
1               5                   10                  15
```

<210> 3
<211> 12
<212> PRT
<213> Pleurotus floridanus

<400> 3

```
Thr Val Gln Thr Asn Ala Pro Trp Gly Leu Ser Arg
1               5                   10
```

<210> 4
<211> 16
<212> PRT
<213> Pleurotus floridanus

<400> 4

```
Ala Pro Ser Ala Leu Thr Val Gly Ala Ser Thr Leu Thr Asp Thr Arg
1               5                   10                  15
```

<210> 5
<211> 1161

<212> DNA
<213> Pleurotus ostreatus

<400> 5

```
atgcgtctct tctctgctgt tcttgcatcc ctcgccatct tggctcctgc ctttgccgct 60
cctgctttga agacggtcga gagcttcgcc ggacagcgca acgatggcag tttcatcgtc 120
aagctcaagt ctggcgcatc ccgaagtggg ctcctcaaga ccctgggcgt caacgcaacc 180
cacgagtggg acgcagccct caacggcttt gctggtaaat tctcggagaa ggcgttgaat 240
gctctgcgcg cctcccctga tgtcgagtcc atctcagagg atggtatcat gcacacattc 300
gtcacccaga ccaacgctcc ttggggactg tctcggttga cttccgcgac ccgcctcacc 360
aacacgaacg ttgccgcatt gactttcaca tacacctacg acgcctccgc tggcagtggt 420
gttgatgtct tcgtcgtcga caccggtatc ttcaccagcc actctcagtt cggtggccgt 480
gcccgctggg gtgctacatt tggaccttac gccgatgctg acggcaatgg tcacggtact 540
cattgcgctg gtaccatcgg tggtagccaa tttggtgtcg ccaagagcgt caacctcatc 600
gccgtgaaag ttctcagcga cggtggatct ggctcggttg ctgatatcgt ctctggcctt 660
aacttcgtcc tgtcctctgc tcgctcttct ggccgccctt ccatcgtctc gatgagtctc 720
ggtggtggtg cttccactgc cttggacaac gccgttgctt ctctcacggc gggtggagtc 780
catgtcgtag ttgccgcggg taactctaac gttgatgctg gcaccacctc ccctgctcgt 840
gccccctctg ccatcactgt cggagcatct accatcactg acactcgagc ctctttctct 900
aacttcggct ccgtcgttga tgtcttcgct cctggtcaag atgtcatcag ctcctggatc 960
ggcagcacca ccgcaaccaa cagaatctct ggaacttcca tggctacccc ccatgttgct 1020
ggcttggccg cctacctcat cgccctcaac ggtaactcat cccctgccgc cctatcgacc 1080
accatcaaga gtttgtcctt gaagggtgtc ctgagcggta ttccttcggg cactctcaac 1140
gacttggctc accacgctta a                                           1161
```

<210> 6
<211> 386
<212> PRT
<213> Pleurotus ostreatus

<400> 6

Met Arg Leu Phe Ser Ala Val Leu Ala Ser Leu Ala Ile Leu Ala Pro
1 5 10 15

Ala Phe Ala Ala Pro Ala Leu Lys Thr Val Glu Ser Phe Ala Gly Gln
20 25 30

Arg Asn Asp Gly Ser Phe Ile Val Lys Leu Lys Ser Gly Ala Ser Arg
35 40 45

Ser Gly Leu Leu Lys Thr Leu Gly Val Asn Ala Thr His Glu Trp Asp
50 55 60

Ala Ala Leu Asn Gly Phe Ala Gly Lys Phe Ser Glu Lys Ala Leu Asn
65 70 75 80

Ala Leu Arg Ala Ser Pro Asp Val Glu Ser Ile Ser Glu Asp Gly Ile
85 90 95

Met His Thr Phe Val Thr Gln Thr Asn Ala Pro Trp Gly Leu Ser Arg
100 105 110

Leu Thr Ser Ala Thr Arg Leu Thr Asn Thr Asn Val Ala Ala Leu Thr
115 120 125

Phe Thr Tyr Thr Tyr Asp Ala Ser Ala Gly Ser Gly Val Asp Val Phe
130 135 140

Val Val Asp Thr Gly Ile Phe Thr Ser His Ser Gln Phe Gly Gly Arg
145 150 155 160

Ala Arg Trp Gly Ala Thr Phe Gly Pro Tyr Ala Asp Ala Asp Gly Asn
165 170 175

Gly His Gly Thr His Cys Ala Gly Thr Ile Gly Gly Ser Gln Phe Gly
180 185 190

Val Ala Lys Ser Val Asn Leu Ile Ala Val Lys Val Leu Ser Asp Gly
195 200 205

Gly Ser Gly Ser Val Ala Asp Ile Val Ser Gly Leu Asn Phe Val Leu
210 215 220

Ser Ser Ala Arg Ser Ser Gly Arg Pro Ser Ile Val Ser Met Ser Leu
225 230 235 240

Gly Gly Gly Ala Ser Thr Ala Leu Asp Asn Ala Val Ala Ser Leu Thr
245 250 255

Ala Gly Gly Val His Val Val Val Ala Ala Gly Asn Ser Asn Val Asp
260 265 270

Ala Gly Thr Thr Ser Pro Ala Arg Ala Pro Ser Ala Ile Thr Val Gly
275 280 285

Ala Ser Thr Ile Thr Asp Thr Arg Ala Ser Phe Ser Asn Phe Gly Ser
290 295 300

Val Val Asp Val Phe Ala Pro Gly Gln Asp Val Ile Ser Ser Trp Ile
305 310 315 320

Gly Ser Thr Thr Ala Thr Asn Arg Ile Ser Gly Thr Ser Met Ala Thr
325 330 335

Pro His Val Ala Gly Leu Ala Ala Tyr Leu Ile Ala Leu Asn Gly Asn
340 345 350

Ser Ser Pro Ala Ala Leu Ser Thr Thr Ile Lys Ser Leu Ser Leu Lys
355 360 365

Gly Val Leu Ser Gly Ile Pro Ser Gly Thr Leu Asn Asp Leu Ala His
370 375 380

His Ala
385

## Description

### Field of the invention

The present invention relates to peptidase enzymes derived from the mycelia of Basidiomycetes, in particular to gluten-specific peptidase enzymes, to their method of production and to their use in the hydrolysis of proteins.

### Background of the invention

The hydrolysis of plant proteins represents one of the oldest processes of food biotechnology. Empirically conducted over centuries in Asian countries, soy protein is hydrolysed to produce among others *Shoyu*, a soy sauce and common condiment which in the Western world has become almost synonymous to Chinese cooking. In these tedious, sometimes perennial processes, the required peptidolytic activities are derived from a consecutive fermentation of *Aspergilli*, lactic acid bacteria and yeasts growing in the respective plant substrates.

The Western countries have imitated this process using wheat, rice or peanut proteins and replacing the enzymatic hydrolysis by acid hydrolysis, for example using hydrochloric acid, to efficiently open the kinetically very stable peptide bonds. The products, usually termed hydrolysed vegetable protein (HVP), are either liquid condiments or concentrated to obtain pastes, powders, premixes for the use in soup or sauce compositions, or stock cubes. All of the products impart a meat-like smell and taste, although no meat is involved at any stage of the process.

As the acid has to be neutralised after the hydrolytic process has been completed, sodium hydroxide is added. The sodium chloride formed during this neutralisation reaction supports the taste enhancing properties of the plant hydrolysate and is often welcome in the final product.

However, acid hydrolysis suffers many disadvantages. Strong acids and alkali are dangerous substances and regarded as being no longer compatible with safe and food grade operations, especially at temperatures above 100°C. Even more problematic is the inevitable formation of dichloro compounds, such as 1,2- and 1,3-dichloropropanol, which were shown to be carcinogenic in animal feeding studies. The contact of the lipidic fraction of the raw materials with hydrochloric acid also generates chlorinated steroids, while compounds such as 5-(chloromethyl)furfural are derived from Maillard pathways.

In order to avoid formation of dangerous or toxic compounds, the attention has turned again to enzymatic processes wherein these compounds are neither required nor formed. As a consequence, there has been an immense interest in improving enzymatic processes.

Peptidases are classified either according to their mechanism of action on the protein substrate (endo or exopeptidases; exopeptidases may be amino or carboxylpeptidases) or to one of the four catalytic mechanisms. Serin-endopeptidases are represented by the chymotrysin and subtilisin superfamilies; they show a broad substrate specificity and contain the so-called catalytic triade. Cystein-endopeptidases are represented by four superfamilies and occur in microbial, plant and animal sources; the name points to the cystein moiety in the catalytic triade. Aspartic acid-endopeptidases cleave peptide bonds by a concerted action of two aspartate moieties. Metallopeptidases are evolutionary old, usually zinc containing enzymes with exo- and endo specificities.

Common peptidases are, for example, alcalase (a serine-peptidase from *Bacillus licheniformis*), papain (a cystein-peptidase from *Carica papaya*), chymosin (rennet) from recombinant *Mucor* strains and neutrase (a metal-peptidase from *Bacillus subtilis*). US 3694316 describes a process for preparing protease from Basidiomycetes. Other commercial peptidases, such as pronase or Flavorzyme®, are mixtures of various types of activities produced by bacteria such as *Streptomyces griseus* or by ascomycete fungi such as *Aspergillus oryzae.*

Due to its biocatalytic nature, the enzymatic process can be performed at gentle physical conditions and is thus more environmentally friendly than its acid catalysed counterpart. However, in contrast to acid hydrolysis, the enzymatic process often does not reach completion and may result in partially hydrolysed products with a bitter taste; the level of bitterness increases with increasing degree of hydrolysis to reach a maximum and then falls with progressing hydrolysis.

Numerous attempts have been published to accelerate the enzymatic process (Pedroche, J. et al., Electronic Journal of Environmental, Agricultural and Food chemistry, 2003) and to avoid the formation of bitter peptides (Maehashi K. et al., Molecular Methods of plant Analysis, 2002, 47-68).

Proteins of plants (Lamsal, B.P. et al., Journal of the American Oil Chemists' Society, 83(8), 731-737, 2006) or animal origin (Je, J. et al., European Food Research and Technology, 221(1-2), 157-162, 2005), or processing wastes such as rice bran have served as substrates (Jarunrattanasri, A. et al., ACS Symposium Series, 83-97, 2005). Substrates such as hemoglobin, casein and insulin have also been investigated (Suzuki, N. et al. Phytochemistry, 2005, 66, 983-990).

Enzymes from many microbial sources, even from pathogenic species (Moreira, F.G. et al., Brazilian Journal of Microbiology, 36(1), 7-11, 2005), have been suggested. For instance, an aspartic peptidase from *Pleurotus eryngii* was found to be active towards casein (Wang, H. et al. Biochem. Biophys. Res. Commun. 2001, 289, 750-755). Metallopeptidases from *Grifola frondosa* and *Pleurotus ostreatus* have been characterised and mechanistically evaluated (Nonaka, T. et al., J. Biol. Chem., 1997, 272, 30032-30039; Hori, T. et al., Acta Crystallogr. D Biol. Chrystallogr., 2001, 57, 361-368). EP1074632 also relates to the enzymatic hydrolysis of protein using fungal cultures.

Combinations of hydrolytic activities have been suggested to show synergistic properties (Minones Conde, J. et al., Journal of Agricultural and Food Chemistry, 53(20), 8038-8045, 2005). The presence of a glutaminase activity was important to obtain a hydrolysate with an intense and typical taste (Schlichtherle-Cerny, H. et al., Journal of Agricultural and Food Chemistry, 50(6), 1515-1522, 2002 and EP 1290951). US3912822 relates to a process for producing a protein hydrolysate having a particularly high glutamic acid content.

However, only very few patents give details on the chemical composition of the products of hydrolysis (US 6465209) or on the type of peptide bonds cleaved preferably (JP 01291795).

Plant enzymes are particularly suited to degrade a plant substrate; to use the same plant as a source of protein and enzyme was an intriguing idea (Tchorbanov, B., Special Publication - Royal Society of Chemistry, 491-494, 2001).

Soy (WO 2002/069733; WO 2004/056980) and wheat (WO2006/104022, Abe, M. et al. Biosci. Biotechnol. Biochem., 2003, 67, 2018-2021) are still the most common enzymatic hydrolysis substrates, while the hydrolysis of animal proteins is often an attempt to minimise side streams of food processing, such as fish bones or blood (JP 2005176620; RU2003-106447). Among the micro-organisms used are combinations of bacteria (WO2002/085131), mixtures of the traditional species (JP2006/075006), recombinant *Aspergillus* (JP2005/328738), or a basidiomycete (JP2006/094757). Sabotič, J. *et al*. in J. Biotechnology, 128, p.297-307, 2006, report on the peptidolytic diversity of European Basidiomycetes. If the biocatalyst was not an intact cell, single peptidases, such as neutrase (WO2006/103628), or combinations of plant and microbial enzymes (WO2002/078461; CN1397644) were suggested.

Mixtures of hydrolytic activites are also helpful, if a high degree of hydrolysis is aimed at (WO2001/076391; WO2003/061675; WO2002/001961). A yeast extract rich in 5'-nucleotides was produced in a sequential process using enzymes from Actinomycetes and then a Basidiomycete, *Trametes sanguinea* (JP2004/229540). A Lactobacillus strain was claimed to provide a glutaminase with specific properties to enhance the yield of glutamate (EP1290951).

Peptidolysis of wheat gluten in particular has been achieved using alcalase, pancreatin or pepsin (Kong, X. et al., Food Chemistry, 101, 615-620, 2007), or papain (Wang, J. et al., Food Chemistry, 101, 1658-1663, 2007). Finally, it is also known to hydrolyse wheat gluten enzymatically using commercially available enzymes. The critical points in the development of WGH-te (Wheat Gluten Hydrolysatetechnical enzymes) processes however are mainly the high costs of these enzymes, separation of solids from the hydrolysates with high yield and microbiological protection of the hydrolysates since they are run without or at low salt concentrations, at temperatures permissible for certain spoilage micro-organisms.

## Object of the invention

The object of the present invention is thus to provide an alternative source of peptidase enzymes which overcome at least some of the inconveniences described above.

## Summary of the invention

Accordingly, the present invention provides, in a first aspect, a method for producing hydrolysates of protein and/or fractions thereof, comprising the steps of:

a. Providing a mixture of peptidases obtained from mycelia of Basidiomycetes,
b. Adding the mixture to a substrate containing said protein and/or fractions thereof and
c. Performing the hydrolysis of said protein and/or fractions thereof to obtain said hydrolysates;

wherein the mixture of peptidases comprises at least one peptidase comprising the sequence given in SEQ ID Nos 1, 2, 3 or 4.

A second aspect of the invention relates to the use of a mixture of peptidases obtained from mycelia of Basidiomycetes for the hydrolysis of gluten and/or fractions thereof, wherein the mixture of peptidases comprises at least one peptidase comprising the sequence given in SEQ ID Nos 1, 2, 3 or 4.

The use of hydrolysates obtained by a method of any of claims 1 to 7 in a food composition, a cosmetic composition and/or a pharmaceutical or medical composition also forms part of the present invention.

The present invention relates, in further aspects, to a mixture of peptidases, wherein said mixture is produced by mycelia of Basidiomycetes, preferably of Hymenomycetes, and wherein at least one peptidase comprises the sequence given in SEQ ID Nos 1, 2, 3 or 4.

The mixture of peptidases has a substrate specificity towards gluten and/or fractions thereof.

**Figures**

The present invention is described hereinafter with reference to some embodiments shown in the figures wherein:

- **Fig. 1** shows silver stained gel (A) and activity stained gel (B) of the proteins (A) and peptidases (B) secreted by *Agrocybe aegerita* during eight days of cultivation.
- **Fig. 2** is an activity stained gel of peptidases of *Pleurotus eryngii* grown on gluten fractions larger 100 kDa.
- **Fig. 3** is an activity stained gel of peptidases of *Grifola frondosa* grown on pure gluten.
- **Fig. 4** shows the pH optima of the peptidase mixtures secreted by six basidiomycetes species.
- **Fig. 5** shows the temperature optima of the peptidase mixtures secreted by six basidiomycetes species.
- **Fig. 6** is an IEF (isoelectic focussing gel electrophoresis) of secretome (A) and peptidases (B) of *Pleurotus sapidus* (lane 2), *Pleurotus eryngii* (lane 3), *Grifola frondosa* (lane 4), and *Lentinula* edodes (lane 5).
- **Fig. 7** shows the evaluation of the mechanism of catalysis of the peptidase of *Pleurotus sapidus* using activity stain in the presence of inhibitors.
- **Fig. 8** is a graph showing the free protein released by the peptidases of *Pleurotus floridanus* (Pfl) and *Grifola frondosa* (Gfr).
- **Fig. 9** is a graph showing the formol number of hydrolysates produced using crude peptidases of *Pleurotus floridanus* (Pfl) and *Grifola frondosa* (Gfr).

**Detailed description of the invention**

In the present application, the term peptidase and protease are used interchangeably and designate any enzyme capable of cleaving one or more peptide bonds and thus, capable of hydrolysing proteins to varying degrees.

The present invention relates to the production of hydrolysates of protein and/or fractions thereof. In a first step, a mixture of peptidases obtained from mycelia of Basidiomycetes is provided.

The division *Basidiomycota* is a large taxon within the Kingdom Fungi that includes species that produce spores in a club-shaped structure called a *basidium*. The Basidiomycota are grouped into three major classes, the Hymenomycotina (Hymenomycetes; mushrooms), the Ustilaginomycotina (Ustilaginomycetes; true smut fungi), and the Teliomycotina (Urediniomycetes; rusts).

Preferably, the Basidiomycetes used in the process of the invention are selected from Hymenomycetes. More preferably, they are selected from the genera *Pleurotus, Lentinula, Agrocybe*, or *Grifola.* Even more preferably, the Basidiomycetes is selected from strains of *Pleurotus sapidus, Pleurotus eryngii, Pleurotus ostreatus, Lentinula edodes, Agrocybe aegerita, Pleurotus floridanus*, or *Grifola frondosa.* Such strains are commercially available through commercial culture collections, such as the DSMZ (Deutsche Sammlung fur Mikroorganismen und Zellkulturen GmbH, Braunschweig, Germany).

Most of these mushrooms typically grow on dead or rotting wood. Thus, they are famous for their potent cellulase (brown rot fungi) and lignin degrading capabilities (white rot fungi). Typical enzymes of white rot fungi are lignin peroxidases, manganese peroxidases, laccases, and some $H_2O_2$ producing oxidases. As deciduous and coniferous wood does not contain a significant amount of protein, it is surprising to find high peptidase activity in mushrooms growing in this particular natural habitat.

According to the invention, the mixture of peptidases is obtained from the mycelia of said micro-organisms. The use of mycelia offers advantages in terms of ease of production and cultivation as the bulk of fruiting bodies is avoided. Also as a result of the extensive use of these species as foodstuffs, the toxicological risks are negligible.

These fungi can be grown in submerged cultures with minimum demands for medium supplements. A carbon, a nitrogen, and a phosphorous source have to be present; a common mixture of minor and trace elements as recommended in all nutrient media of micro-organisms is preferably added. This process of biomass production is operated under mild conditions and is environmentally friendly.

The temperatures are moderate, typically in the range of from 10 to 35°C, preferably from 20 to 30°C. A medium pH of about 4 to 8, with a pH of about 5 to 7 being preferred, and conditions of low light are typical of the process.

The peptidolytic activities are secreted into the mycelium free culture supernatant, which facilitates the handling of the bioprocess as well as enzyme isolation and enrichment using techniques known in the art, such as centrifugation, ultra-filtration or precipitation. A cell-free, concentrated culture supernatant of peptidases may thus be obtained and further used for hydrolysis. Although the enzymes may be isolated by techniques known in the art, it is not necessary to do so and the crude mixture obtained may also be further used in the present method.

The mixture of peptidases obtained from the mycelia of Basidiomycetes comprises at least one peptidase having the partial sequence as listed in sequence listing (1), (2), (3) or (4).

(1): DGNASPASLSTK
(2) : QLSGLPSGTLNDLAR
(3): TVQTNAPWGLSR
(4): APSALTVGASTLTDTR

In a second step of the method of the present invention, the mixture of peptidases is added to a substrate containing protein and/or fractions thereof. The mixture of peptidases may be that obtained from one micro-organism strain described above or may be a mixture of peptidases obtained from more than one of the micro-organism strains described above. The protein may be selected from any animal or vegetal sources and may be gluten, whey, soy etc. It may be derived from wheat, wheat flour, soy (or press cake), lupines, yellow beans, potatoes, corn, barley, lentils, milk etc. Preferably, it is derived from a plant source. More preferably, it is derived from cultivars of Triticum L. (wheat). Most preferably, the protein is gluten or fractions thereof.

The peptidases are usually applied in the protein substrate dispersion at a total level of from 0.0005 to 5%, preferably 0.0001% to 2% of pure enzyme by weight depending on the specific activity. The enzymes can be added as separate single ingredients (pills, granulates, stabilized liquid, etc. containing one enzyme) or as mixtures of two or more enzymes (e.g. cogranulates).

In a final step, the hydrolysis of said protein and/or fractions thereof is performed to obtain said hydrolysates. The conditions which may be used for the hydrolysis of protein are standard conditions and can be easily determined by a person of skill in the art. For instance, the reaction may include the presence of supporting catalytic activities be they from micro-organisms or other enzyme sources.

Figure 1A shows the presence of a mixture of protein (e.g. peptidases) after cultivation of mycelia of *Agrocybe aegerita* and figure 1B shows the enzymatic activity of said peptidases.

The hydrolysates obtained by a method of the present invention may be peptides of varying size as well as single amino acids. They can be further processed as a liquid. In particular, the solution may be submitted to de-coloration and filtration steps. The refined liquid may then be concentrated to any degree according to the desired form of application. All of these HVP products impart a pleasant savoury and meaty flavour.

The hydrolysates may be used in food compositions, cosmetic composition and/or pharmaceutical or medical compositions.

Thus, the compositions may be beverages, cheese, confectionery, desserts, dressings, fruit drinks, meat products, snacks, nutritional supplements, feeds, pet food supplements, medical diets, skin or hair products, fertilisers, veterinary preparations, constituents for the *in vitro* cultivation of mammalian cells etc.

Additionally, the hydrolysates may be used as emulsifiers, foam stabilisers, water binding agents etc. When used in food, the hydrolysates obtained according to the invention may be used as food flavour or food flavour precursor.

Thus, the use of a mixture of peptidases obtained from mycelia of Basidiomycetes for the hydrolysis of protein, and in particular gluten and/or fractions thereof is part of the present invention.

The peptidases of the present invention catalyse the hydrolysis of proteins, in particular gluten, to larger and smaller peptides and finally to amino acids. While the intact proteins do not impart a taste or smell to food, the free amino acids obtainable with the method according to the present invention are very useful as flavour compounds and flavour precursors for the use in soups, sauces and many kinds of convenient foods. In addition, the peptidase treatment as mentioned can be combined with another enzyme which cleaves different peptide bonds to further improve the flavour and/or fragrance of the food products.

The degree of hydrolysis can be either assessed visually or using analytical chemistry. The gluten substrate is a soft, sticky material resembling chewing gum. After incubation in a peptidase containing buffer the material shows typical signs of degradation, i.e. it turns into a turbid dispersion with some larger and many fine and very fine particles.

Hydrolysis can also be measured by the determination of free protein in the solution before and after peptidolysis (Figure 8), by measuring the free amino-Nitrogen in solution (Kjeldahl or formol number (Figure 9)), by following protein degradation using SDS-PAGE (sodium dodecyl sulfate polyacryamide gel electrophoresis), or by thin layer chromatography followed by Ninhydrin detection.

The degree of hydrolysis for the mixture of peptidases of the present invention as determined by the formol number may range from 3 to 20 DH, preferably from 6 to 19 DH; the total soluble protein in the dispersion may range from at least 10%, preferably from 19 to 80%.

Another aspect of the invention relates to a method for the production of a mixture of peptidases comprising the step of cultivating mycelia of Basidiomycetes in a growth medium comprising gluten and/or fractions thereof and isolating the mixture of peptidases from the culture.

Preferably, gluten and/or fractions thereof is the principal source of nitrogen in the growth medium.

When gluten fractions are used in the present method, these may be greater than 100kDa, between 5 and 100kDa or processed gluten hydrolysates. The culture may contain a mixture of any of these gluten fractions. These may be obtained commercially or produced by methods known to the skilled person.

Preferably, the Basidiomycetes mycelia used in the present process are selected from Hymenomycetes. More preferably, they are selected from the genera *Pleurotus, Lentinula, Agrocybe,* or *Grifola.* Even more preferably, the Basidiomycetes is selected from strains of *Pleurotus sapidus, Pleurotus eryngii, Pleurotus ostreatus, Lentinula edodes, Agrocybe aegerita, Pleurotus floridanus,* or *Grifola frondosa.* Such strains are commercially available through commercial culture collections, such as the DSMZ (Deutsche Sammlung für Mikroorganismen und Zellkulturen GmbH, Braunschweig, Germany).

Preferably, the cultivating is carried out in a submerged culture, for 1 to 15 days, preferably for 3 to 12 days.

After cultivation, the mixture of peptidases produced is isolated from the culture. It can further be concentrated by precipitation and/or centrifugation.

Thus, the mixture of peptidases obtainable by the present method also forms part of the present invention. The advantage provided by said method is that the resulting mixture of peptidases has a distinct affinity, and improved efficacy in the hydrolysis of gluten. The mixture of peptidases according to the invention has a substrate specificity towards gluten and/or fractions thereof.

Even more surprising are the excellent properties of these enzymes with regard to converting gluten to smaller peptides and finally to the single amino acids. Crude mixtures of the peptidase enzyme are capable of very efficiently and gently hydrolysing gluten fractions of 5 to 100 kDa (Figure 1), gluten fractions larger than 100 kDa (Figure 2), and native untreated gluten (Figure 3). No additive or co-substrate other than water is necessary. The incubation medium is simple and may contain, for example, 0.5 g $MgSO_4$ $L^{-1}$, 1.5 g $KH_2PO_4$ $L^{-1}$, 0.4% Tween 80 and a trace element solution.

The novel peptidases possess a broad temperature optimum between 40 and 60°C and a likewise broad pH-optimum between pH 4.5 and 7, as determined by the classical azocasein assay (Figure 4 and 5). The isoelectric points cover a range from 4 to 10, and the molecular masses of the various isoenzymes ranges from below 30 kDa to more than 100 kDa, as determined using IEF (isoelectic focussing gel electrophoresis) and native polyacrylamide gel electrophoresis (PAGE) (Figures 1, 2, 3 and 6). Specific inhibition studies have shown that most of the isoenzymes are serine and aspartate petidases, but some isoenzymes could not be assigned to one of the classical types (Figure 7).

The unique partial sequences of two representatives of the novel gluten induced peptidases were determined by ESI-MS analyses (Table 1). The best homologies were found to serine peptidases of *Agaricus bisporus* (Champignon). Thus, the classification of the novel enzymes to the peptidases, a sub-class of the hydrolases (EC 3.4.X.X) was confirmed.

**Table 1** Partial sequences of peptidases of *Grifola frondosa* (Gfr) and *Pleurotus floridanus* (Pfl)

| | [kDa] | homologous to | e |
|---|---|---|---|
| Gfr | 29 | | |
| DGNASPASLSTK | | serine proteinase (A. bisporus) | 16 |
| QLSGLPSGTLNDLAR | | signal peptide peptidase | 50 |
| Pfl | 28 | | |
| TVQTNAPWGLSR | | serine proteinase (A. bisporus) | 1.6 |
| APSALTVGASTLTDTR | | serine proteinase precursor (A. bisporus) | 16 |

The present invention is described further herein by way of illustration in the following non-limiting examples.

## Examples

In the following examples, the following materials and methods were used.

## Materials and Methods

### Concentration of culture supernatant

To concentrate the secreted peptides, the culture supernatant was precipitated with ethanol in a total concentration of 80% for 3 hours at -20 °C. The precipitate was centrifuged at 4 °C for 20 min at 12,000xg and stored at -20 °C.

### Biochemical enzyme characterisation

The biochemical characterisation of the peptidases comprised the determination of the pH and temperature optima, the isoelectric point by IEF with activity staining (Figure 6), and a molecular weight analysis by SDS-PAGE.

### SDS-PAGE and native PAGE

SDS-PAGE analyses were performed on a 12% polyacrylamide separation gel. Samples were prepared by mixing 20 μL of enzyme solution and 20 μL of loading buffer [0.1 M Tris/HCl (pH 6.8), 0.2 M DTT, 4% SDS, 20% glycerol, 0.2% bromophenol blue] and boiling for 15 min. After electrophoresis at 20 mA per gel, the gels were silver or Coomassie-stained. For molecular determinations, marker proteins from 250 to 10 kDa (BioRad, Germany) were used. Native PAGE was performed under non-denaturating conditions. The separation gel contained 0.04% porcine gelatine. Samples were prepared by mixing 1:1 (v/v) with loading buffer [0.05 M Tris/HCL (pH 6.8), 2% SDS, 10% glycerol, 0.1% bromophenol blue]. After electrophoresis at 10 mA per gel and at 8°C, gels were washed 2 times in 2.5% Triton X-100. After incubation in 0.1 M $K_2HPO_4/KH_2PO_4$ buffer (pH 6) for 14 to 18 hours at 30°C the gels were Coomassie-stained. Enzyme activity appeared as white bands on a blue background.

### Isoelectric focusing

IEF polyacrylamide gel electrophoresis was performed on a Multiphor II system (Pharmacia LKB, Schweden) using Servalyt™ Precotes™ precast gels with an immobilised pH gradient from 3 to 10 (Serva, Germany) for 3500 V h (2000 V, 6 mA, 12 W). The isoelectric points of the peptidases were 5 estimated using a protein test mixture from pI 3.5 to 10.7 (Serva, Germany). Gels were Coomassie, silver or activity stained. For activity staining, a porcine gelatine agarose-overlay gel was developed. Agarose (2%) was dissolved in 0.1 M $K_2HPO_4/KH_2PO_4$ buffer (pH 6) under boiling. Equivalent volumes of 0.04% porcine gelatine in 0.1 M $K_2HPO_4/KH_2PO_4$ buffer (pH 6) were added. After focusing, the IEF gel was incubated on the overlay at 30 °C for 14 to 18 hours, and the overlay gel was Coomassie stained. Enzyme activity appeared as white bands on a blue background.

### Activity test

Substrate solution consisted of 0.5% of azocasein in 0.1 M $K_2HPO_4/KH_2PO_4$ buffer at pH 6. Enzyme activity was measured at 37 °C in a total volume of 2 mL containing 100 μL of substrate and 300 μL of $K_2HPO_4/KH_2PO_4$ buffer (0.1 M, pH 6). The reaction was started with the addition of 100 μL of enzyme solution.
Depending to the enzyme activity the reaction was stopped after 15 to 30 min with 1.5 mL of trichloric acetic acid (3%). The samples were stored on ice for 10 min and centrifuged at 11,600xg for 10 min. The absorbance of the supernatant was measured at 366 nm using a spectral photometer. A blank was prepared with heat inactivated enzyme solution. Enzyme activity was calculated according to the following equation

$$A\ (U\ mL^{-1}) = \frac{\Delta E60}{0.01 V_s t}$$

where U is the enzyme activity catalysing the hydrolysis of azocasein expressed as the difference in absorbance per hour. ΔE is the difference in absorbance between blank and sample, $V_s$ is the sample volume (mL), and t is the incubation time (min).

### Temperature and pH optima

The determination of the temperature and pH optima of the secreted peptidases was performed with enzyme solutions harvested at the cultivation day of maximum activity. First, the temperature optima (Figure 5) was performed at pH 6.0 with 0.1 M $K_2HPO_4/KH_2PO_4$ buffer as described above. At an optimal temperature of 50 °C the pH variations ranged from 4.5 to 8.0 (Figure 4).

**Catalytic mechanism**

To determine the catalytic mechanism, native PAGE analysis was performed as aforementioned with modifications in the sample preparation techniques. The enzyme solution was mixed with 1 mM PMSF (phenylmethyl sulfonyl fluoride), 10 mM EDTA (ethylenediaminetetraacetic acid), 1 mM aprotinin, and 1 mM pepstatin A, respectively. The samples were then incubated for 30 min at 4 °C prior to electrophoresis.

**ESI-Tandem MS analysis of tryptic peptides**

The peptidase bands were excised from SDS polyacrylamide gels, dried, and digested with trypsin. The resulting peptides were extracted and purified according to standard protocols. A Qtof II mass spectrometer (Micromass, U.K) equipped with a nanospray ion source and gold-coated capillaries was used for electrospray ionisation (ESI) MS of peptides. For collision-induced dissociation experiments, multiple charged parent ions were selectively transmitted from the quadrupole mass analyser into the collision cell (25-30 eV). The resulting daughter ions were separated by an orthogonal time-of-flight mass analyser. Peptide mass fingerprints obtained from ESI-Tandem MS analysis were used for cross-species protein identification in public protein primary sequence databases (Table 1).

**Hydrolysis experiments**

0.5 g of gluten were suspended in 20 mL of 0.1 M $K_2HPO_4/KH_2PO_4$ buffer (pH 6.0). After the addition of 18.9 kU of enzyme preparation, the sample was incubated for 18 h at 40 and 50 °C, respectively, at 110 rpm in a water bath, and was centrifuged at 3000xg for 15 min at 4°C. The hydrolysis was stopped by storing the supernatant at 4 °C. The following analysis methods were used to estimate the degree of hydrolysis.

**Free protein**

The protein concentration in the hydrolysis supernatant was estimated according to the *Lowry*-method using bovine serum albumin as a standard.

**Formol number**

The titration of the formol number in the hydrolysis supernatant was performed on a Titroline easy (Schott, Germany). 3 to 10 mL of the experimental solution were adjust to pH 8.1 with 0.1 M NaOH. Then 10 mL of formalin solution (pH 8.1, 35-37%) were added. After 1 min the sample was titrated with 0.1 M NaOH at pH 8.1. The formol number was calculated according to the following equation

$$\text{Formol number} = \frac{V_{NaOH}\ 100}{V_S}$$

where $V_s$ is the sample volume (mL), and $V_{NaOH}$ is the titrated volume of 0.1 M NaOH (mL).

**Kjeldahl analysis**

The determination of nitrogen in the hydrolysis supernatant was performed on a Digestion Unit K-424 and a distillation unit K-350 (Büchi, Switzerland) according to standard protocols. The ammonia of 1 to 5 mL of experimental solution was captured by an excess of 0.1 M HCl in the receiving flask. The back titration of non-consumed volume of the acid solution was neutralised by 0.1 M NaOH using a Titroline easy titrator. The amount of nitrogen was calculated according to the following equation

$$\text{nitrogen (\%)} = \frac{(V_{HCl}\text{-}V_{NaOH})\ 0.1\ 20\ 100}{V_S\ n_{gluten}}$$

where VS is the sample sample (mL), and ngluten is the total amount of nitrogen (4.62 mmol) in 0.5 g gluten.

**Amino acid thin layer chromatography (AA-TLC)**

For TLC separation, 7 μL of the hydrolysis supernatant were spotted on an aluminium plate coated with silica gel (20 cm x 20 cm). The plate was then placed in a chamber with n-butanol/acetic acid/water (70:20:30 v/v/v) for 4 h. Detection of the amino acids was carried out by spraying Ninhydrin reagent (0.4 g 100 mL-1 acetone) followed by an incubation at 100 °C until the coloured spots appeared. The amino acids generated were identified by comparison with reference compounds.

<u>Example 1 - Peptidases from six Basidiomycetes.</u>

**Cultivation of basidiomycetes**

All media and equipment were autoclaved prior to use and standard sterile techniques were applied throughout the procedure. Pre-cultures of basidiomycetes (Table 2) were grown for seven days in standard nutrient medium (30 $g.L^{-1}$ glucose monohydrate, 3 $g.L^{-1}$ yeast extract, 4.5 $g.L^{-1}$ asparagine monohydrate, 0.5 $g.L^{-1}$ $MgSO_4$, 1.5 g. $L^{-1}$ $KH_2PO_4$, 1 mL trace element solution, pH 6.0 [0.08 $g.L^{-1}$ $FeCl_3$, 0.09 $g.L^{-1}$ $ZnSO_4$ x 7 $H_2O$, 0.005 $g.L^{-1}$ $CuSO_4$ x 5 $H_2O$, 0.027 g. $L^{-1}$ $MnSO_4$, 0.4 $g.L^{-1}$ Titriplex III]) at 150 rpm and 24°C.

20 mL of the mycelium culture were separated by centrifugation (10 min, 3000xg, 4 °C) and washed 2 times with 20 mL of mineral salt medium (MM) (0.5 $g.L^{-1}$ $MgSO_4$, 1.5 $g.L^{-1}$ $KH_2PO_4$, 1 mL trace element solution, pH 6.0), lacking an additional carbon and nitrogen source. For the main cultures, 250 mL of fresh MM were inoculated with 20 mL of the prewashed mycelia.

**Table 2** Basidiomycetes cultivated for the enzyme screenings.

| Strain | | Source |
|---|---|---|
| *Pleurotus sapidus* | Psa | DSMZ 8266 |
| *Pleurotus eryngii* | Per | DSMZ 366.47 |
| *Grifola frondosa* | Gfr | DSMZ 480.63 |
| *Pleurotus floridanus* | Pfl | Göttingen No. 17 |
| *Agrocybe aegerita* | Aae | Sylvan 4021 |
| *Lentinula edodes* | Led | A 20-5 |

**Enzyme induction**

To induce enzyme secretion into the growth medium, sterile hydrolysate fraction from 5 to 100 kDa, fraction larger than 100 kDa, and process hydrolysate, respectively, were added to the main cultures. In case of gluten induction MM with 4% of gluten was prepared. After 3 to 12 days, the mycelium was separated by filtration and the extracellular enzyme-containing supernatant was employed for the enzymatic conversions.

<u>Example 2 - Enzyme preparation using *Grifola fondrosa*</u>

After 6 days of cultivation with 4% gluten, the *Gfr*-culture was filtrated and the extracellular enzyme-containing supernatant (200 mL) was precipitated with 800 mL of ethanol (96%), was stored for 3 hours at -20 °C, and was centrifuged at 12,000xg for 20 min. An aliquot of the pellet was re-suspended in 0.1 M $K_2HPO_4/KH_2PO_4$ buffer (pH 6.0) for 30 min at 4 °C and the activity of the supernatant was measured.

Most of the hydrolytic activity originally present was recovered indicating that this protocol yielded a useful enzyme concentrate.

<u>Example 3 - Hydrolysis experiments</u>

0.5 g of gluten were suspended in 20 mL of 0.1 M $K_2HPO_4/KH_2PO_4$ buffer (pH 6.0), 18.9 kU of the peptidase pellet obtained from *Pfl* (Pleurotus floridanus) was added, and the sample was incubated for 18 h at 40°C at 110 rpm in a water bath. It was centrifuged at 3000xg for 15 min at 4°C. A blank was prepared with heat inactivated enzyme preparation. 5 mL of the supernatant were applied to the Kjeldahl analysis, and 7 μL to the AA-TLC. To visualise the protein hydrolysis, SDS-PAGE of the samples was performed.

This analytical evidence indicated a progressive enzymatic hydrolysis of the difficult substrate, wheat gluten.

Example 4 - Hydrolysis experiments with gluten

0.5 g of gluten were suspended in 20 mL of 0.1 M $K_3HPO_4/KH_2PO_4$ buffer (pH 6.0), 18.9 kU of the peptidase pellet obtained from *Gfr* was added, and the sample was incubated for 18 h at 40°C at 110 rpm in a water bath. It was centrifuged at 3000xg for 15 min at 4°C. A blank was prepared with heat inactivated enzyme preparation. 3 mL of the supernatant were applied to the formol titration, and 20 μL to the protein determination. To visualise the protein hydrolysis, SDS-PAGE of the samples was performed. This confirmed the enzymatic hydrolysis of gluten by an alternative analytical method.

Example 5 - Hydrolysis experiments with combined enzyme preparation

0.5 g of gluten were suspended in 20 mL of 0.1 M $K_2HPO_4/KH_2PO_4$ buffer (pH 6.0), 18.9 kU of *Gfr* and *Pfl* peptidase pellet were added, and the sample was incubated for 18 h at 40°C at 110 rpm in a water bath. It was centrifuged at 3000xg for 15 min at 4°C. A blank was prepared with heat inactivated enzyme preparation. 5 mL of the supernatant were applied to the Kjeldahl analysis, 3 mL to the formol titration, 7 μL to the AA-TLC, and 20 μL to the protein determination. To visualise the protein hydrolysis, SDS-PAGE of the samples was performed.

The use of a mixture of enzymes hence resulted in a rapid hydrolysis of gluten.

Example 6 - Hydrolysis experiments with fraction > 100 kDa

250 μL of a protein fraction >100 kDa were suspended in 2 mL of 0.1 M $K_2HPO_4/KH_2PO_4$ buffer (pH 6.0), 1.9 kU of a peptidase pellet of the present invention were added, and 30 the sample was incubated for 18 h at 50 °C at 110 rpm in a water bath. It was centrifuged at 3000xg for 15 min at 4 °C. A blank was prepared with heat inactivated enzyme preparation. 2 mL of the supernatant were applied to the formol titration, 7 μL to the AA-TLC, and 20 μL to the protein determination. To visualise the protein hydrolysis, SDS-PAGE of the samples was performed.

This confirmed that the novel enzymes were useful for the degradation (hydrolysis) if even non-soluble protein matter.

Example 7 - Hydrolysis experiments with native process hydrolysate

250 μL of the native process hydrolysate were suspended in 2 mL of 0.1 M $K_2HPO_4/KH_2PO_4$ buffer (pH 6.0), 1.9 kU of a peptidase pellet of the present invention were added, and the sample was incubated for 18 h at 50 °C at 110 rpm in a water bath. It was centrifuged at 3000xg for 15 min at 4 °C. A blank was prepared with heat inactivated enzyme preparation. 2 mL of the supernatant were applied to the formol titration, 7 μL to the AA-TLC, and 20 μL to the protein determination. To visualise the protein hydrolysis, SDS-PAGE of the samples was performed.

This confirmed that the novel enzymes were useful for the degradation (hydrolysis) of native hydrolysate as it occurs during the manufacturing process.

Example 8 - Aroma extraction and GC analysis

After hydrolysis of any one of the gluten fractions or the pure gluten, the sample was centrifuged at 3000xg for 15 min at 4 °C, and the supernatant was extracted 3 times with the equivalent volume of pentane/ether. The collected organic fractions were dried and concentrated. 1 μL of the pre-prepared sample, which exhibited a savoury smell, was injected into the GC, and the presence of volatile flavours was proven using a flame ionisation detector.

Thus, the described process yielded a product possessing aroma.

## Claims

**1.** Method for producing hydrolysates of protein and/or fractions thereof, comprising the steps of:

a. Providing a mixture of peptidases obtained from mycelia of Basidiomycetes cultivated in a submerged culture,
b. Adding the mixture to a substrate containing said protein and/or fractions thereof and
c. Performing the hydrolysis of said protein and/or fractions thereof to obtain said hydrolysates;

wherein the mixture of peptidases comprises at least one peptidase comprising the sequence given in SEQ ID Nos 1, 2, 3 or 4.

**2.** Method according to claim 1, wherein the protein is selected from gluten, whey, soy, preferably gluten.

**3.** Method according to claim 1, wherein the peptidases are extracellular enzymes.

**4.** Method according to any of claims 1 to 3, wherein the Basidiomycetes is selected from Hymenomycetes.

**5.** Method according to any of claims 1 to 4, wherein the Basidiomycetes is selected from the genera *Pleurotus,* Lentinula, *Agrocybe,* or *Grifola.*

**6.** Method according to any of claims 1 to 5, wherein the Basidiomycetes is selected from strains of *Pleurotus sapidus, Pleurotus eryngii*, *Pleurotus ostreatus, Lentinula edodes, Agrocybe aegerita, Pleurotus floridanus,* or *Grifola frondosa.*

**7.** Method according to any of claims 1 to 6, wherein the hydrolysates are peptides and/or amino acids.

**8.** Use of a mixture of peptidases obtained from mycelia of Basidiomycetes for the hydrolysis of gluten and/or fractions thereof, wherein the mixture of peptidases comprises at least one peptidase comprising the sequence given in SEQ ID Nos 1, 2, 3 or 4.

**9.** Use of hydrolysates obtained by a method of any one of the claims 1 to 7 in a food composition, a cosmetic composition and/or a pharmaceutical or medical composition.

**10.** Use according to claim 9, wherein the composition is a beverage, cheese, confectionery, desserts, dressing, fruit drinks, meat products, snacks, nutritional supplement, feed, pet food supplement, medical diet, skin or hair product, fertiliser, veterinary preparations, constituent for the *in vitro* cultivation of mammalian cells etc.

**11.** Mixture of peptidases, wherein said mixture is produced by mycelia of Basidiomycetes, preferably of Hymenomycetes, and wherein at least one peptidase comprises the sequence given in SEQ ID Nos 1, 2, 3 or 4.

**12.** Mixture of peptidases according to claim 11, wherein the Basidiomycetes is selected from the genera *Pleurotus*, *Lentinula, Agrocybe,* or *Grifola,* preferably selected from strains of *Pleurotus sapidus, Pleurotus eryngii, Pleurotus*, *ostreatus, Lentinula edodes, Agrocybe aegerita, Pleurotus floridanus,* or *Grifola frondosa.*

**13.** Mixture of peptidases according to claims 11 or 12, said mixture having a conversion efficiency of 3 to 20 DH, preferably 6 to 19 DH.

## Patentansprüche

**1.** Verfahren zur Herstellung von Hydrolysaten aus Protein und/oder Fraktionen davon, das die folgenden Schritte aufweist:

a. Bereitstellen einer Mischung aus Peptidasen, die aus Myzelien von Basidiomyceten gewonnen werden, die in einer Submerskultur gezüchtet wurden,
b. Zufügen der Mischung zu einem Substrat, das das Protein und/ oder Fraktionen davon enthält, und
c. Durchführen der Hydrolyse des Proteins und/oder von Fraktionen davon, um die Hydrolysate zu gewinnen,

wobei die Mischung von Peptidasen mindestens eine Peptidase mit der Sequenz aufweist, die in SEQ ID Nos 1, 2, 3 oder 4 angegeben ist.

**2.** Verfahren nach Anspruch 1, wobei das Protein aus Gluten, Molke, Soja, vorzugsweise Gluten, ausgewählt ist.

**3.** Verfahren nach Anspruch 1, wobei die Peptidasen extrazelluläre Enzyme sind.

**4.** Verfahren nach einem der Ansprüche 1 bis 3, wobei die Basidiomyceten aus Hymenomyceten ausgewählt sind.

**5.** Verfahren nach einem der Ansprüche 1 bis 4, wobei die Basidiomyceten aus den Gattungen *Pleurotus, Lentinula, Agrocybe oder Grifola* ausgewählt sind.

**6.** Verfahren nach einem der Ansprüche 1 bis 5, wobei die Basidiomyceten aus Stämmen von *Pleurotus sapidus, Pleurotus eryngii, Pleurotus ostreatus, Lentinula edodes, Agrocybe aegerita, Pleurotus floridanus oder Grifola frondosa* ausgewählt sind.

**7.** Verfahren nach einem der Ansprüche 1 bis 6, wobei die Hydrolysate Peptide und/oder Aminosäuren sind.

**8.** Verwendung einer Mischung von Peptidasen, die aus Myzelien von Basidiomyceten gewonnen wurde, für die Hydrolyse von Gluten und/ oder Fraktionen davon, wobei die Mischung von Peptidasen mindestens eine Peptidase mit der Sequenz aufweist, die in SEQ ID Nos 1, 2, 3 oder 4 angegeben ist.

**9.** Verwendung von Hydrolysaten, die durch ein Verfahren nach einem der Ansprüche 1 bis 7 gewonnen wurden, in einer Nahrungszusammensetzung, einer Kosmetikzusammensetzung und/oder einer pharmazeutischen oder medizinischen Zusammensetzung.

**10.** Verwendung nach Anspruch 9, wobei die Zusammensetzung folgendes ist: ein Getränk, Käse, Süßwaren, Desserts, Salatsoßen, Fruchtgetränke, Fleischprodukte, Snacks, Nahrungsergänzungsmittel, Futtermittel, Nahrungsergänzungsmittel für Tiere, eine medizinische Diät, ein Haut- oder Haarprodukt, ein Düngemittel, veterinärmedizinische Präparate, Bestandteile für die *in vitro* Kultivierung von Säugerzellen etc.

**11.** Mischung von Peptidasen, wobei die Mischung durch Myzelien von Basidiomyceten, vorzugsweise von Hymenomyceten, hergestellt ist, und wobei mindestens eine Peptidase die in SEQ ID Nos 1, 2, 3 oder 4 angegebene Sequenz aufweist.

**12.** Mischung von Peptidasen nach Anspruch 11, wobei die Basidiomyceten ausgewählt sind aus den Gattungen *Pleurotus, Lentinula, Agrocybe oder Grifola,* vorzugsweise ausgewählt aus Stämmen von *Pleurotus sapidus, Pleurotus eryngii, Pleurotus ostreatus, Lentinula edodes, Agrocybe aegerita, Pleurotus floridanus oder Grifola frondosa.*

**13.** Mischung von Peptidasen nach Anspruch 11 oder 12, wobei die Mischung einen Umwandlungswirkungsgrad von 3 bis 20 DH, vorzugsweise 6 bis 19 DH, hat.

## Revendications

**1.** Procédé pour la production d'hydrolysats de protéine et/ou de fractions de celle-ci, comprenant les étapes consistant à :

a. fournir un mélange de peptidases obtenues à partir de mycéliums de Basidiomycètes cultivés dans une culture submergée,
b. ajouter le mélange à un substrat contenant ladite protéine et/ou lesdites fractions de celle-ci et
c. réaliser l'hydrolyse de ladite protéine et/ou desdites fractions de celle-ci afin d'obtenir lesdits hydrolysats ;

dans lequel le mélange de peptidases comprend au moins une peptidase comprenant la séquence donnée dans SEQ ID Nos 1, 2, 3 ou 4.

**2.** Procédé selon la revendication 1, dans lequel la protéine est choisie parmi gluten, lactosérum, soja, de préférence gluten.

**3.** Procédé selon la revendication 1, dans lequel les peptidases sont des enzymes extracellulaires.

**4.** Procédé selon l'une des revendications 1 à 3, dans lequel les Basidiomycètes sont choisis parmi les Hyménomycètes.

**5.** Procédé selon l'une des revendications 1 à 4, dans lequel les Basidiomycètes sont choisis parmi les genres *Pleurotus, Lentinula, Agrocybe* ou *Grifola.*

**6.** Procédé selon l'une des revendications 1 à 5, dans lequel les Basidiomycètes sont choisis parmi les souches de *Pleurotus sapidus, Pleurotus eryngii, Pleurotus ostreatus, Lentinula edodes, Agrocybe aegerita, Pleurotus floridanus* ou *Grifola frondosa.*

**7.** Procédé selon l'une des revendications 1 à 6, dans lequel les hydrolysats sont des peptides et/ou des acides aminés.

**8.** Utilisation d'un mélange de peptidases obtenu à partir de mycéliums de Basidiomycètes pour l'hydrolyse de gluten et/ou de fraction de celui-ci, dans laquelle le mélange de peptidases comprend au moins une peptidase comprenant la séquence donnée dans SEQ ID Nos 1, 2, 3 ou 4.

**9.** Utilisation d'hydrolysats obtenus par un procédé selon l'une quelconque des revendications 1 à 7 dans une composition alimentaire, une composition cosmétique et/ou une composition pharmaceutique ou médicale.

**10.** Utilisation selon la revendication 9, dans laquelle la composition est une boisson, fromage, confiserie, desserts, assaisonnement, boissons aux fruits, produits à base de viande, collations, complément nutritionnel, nourriture, complément alimentaire pour animal de compagnie, diète médicale, produit pour la peau ou les cheveux, engrais, préparations vétérinaires, constituant pour la culture in vitro de cellules de mammifères etc.

**11.** Mélange de peptidases, dans lequel ledit mélange est produit par des mycéliums de Basidiomycètes, de préférence d'Hyménomycètes, et dans lequel au moins une peptidase comprend la séquence donnée dans SEQ ID Nos 1, 2, 3 ou 4.

**12.** Mélange de peptidases selon la revendication 11, dans lequel les Basidiomycètes sont choisis parmi les genres *Pleurotus, Lentinula, Agrocybe* ou *Grifola,* de préférence choisis parmi les souches de *Pleurotus sapidus, Pleurotus eryngii, Pleurotus ostreatus, Lentinula edodes, Agrocybe aegerita, Pleurotus floridanus* ou *Grifola frondosa*.

**13.** Mélange de peptidases selon les revendications 11 ou 12, ledit mélange présentant un rendement de conversion compris entre 3 et 20 DH, de préférence entre 6 et 19 DH.

Figures

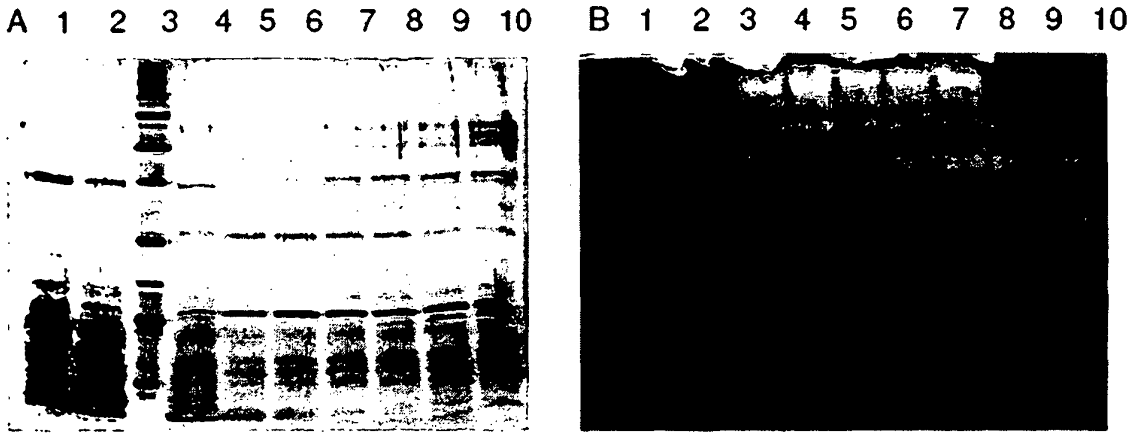


**Figure 1** Silver stained gel (A) and activity stained gel (B) of the proteins (A) and peptidases (B) secreted by *Agrocybe aegerita* during eight days of cultivation of the present invention. Lane 1: cultivation day 0; Lane 2: cultivation day 1; Lane 3: molecular marker; Lane 4 to 10: cultivation day 3 to 8.

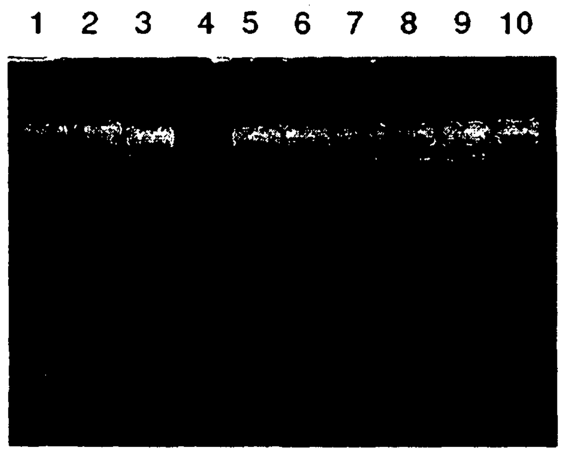


**Figure 2** Activity stained gel of peptidases of *Pleurotus eryngii* grown on gluten fraction larger 100 kDa. Lane 1 to 3: cultivation day 1 to 3; Lane 4: molecular marker; Lane 5 to 10: cultivation day 4 to 9.

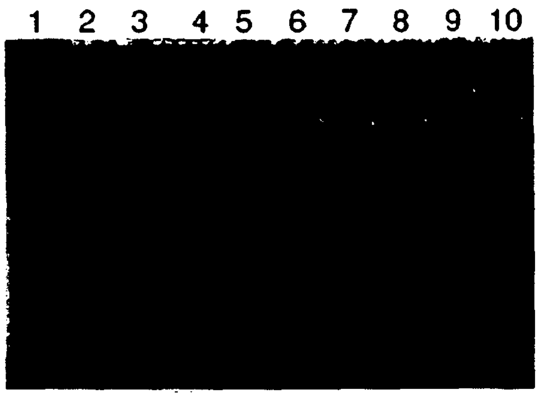


**Figure 3** Activity stained gel of peptidases of *Grifola frondosa* grown on pure gluten. Lane 1: cultivation day 0; Lane 2: molecular marker; Lane 3 to 10: cultivation day 1 to 8.

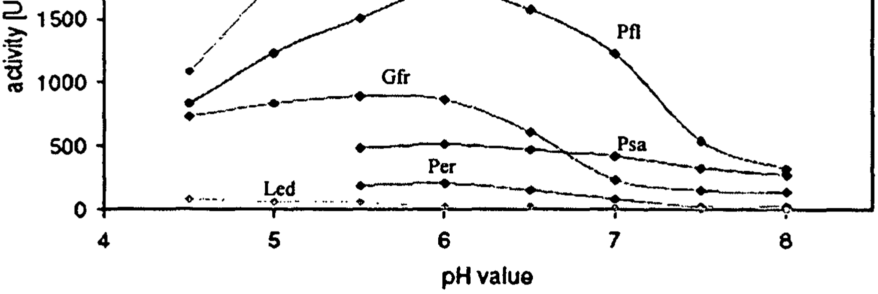


**Figure 4** Determination of the pH optima of the peptidase mixtures secreted by the six basidiomycetes species.

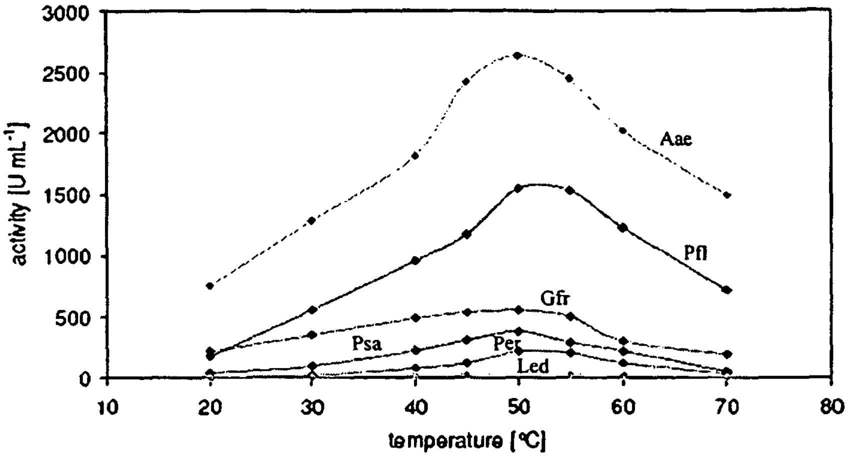


**Figure 5** Determination of the temperature optima of the peptidase mixtures secreted by the six basidiomycetes species.

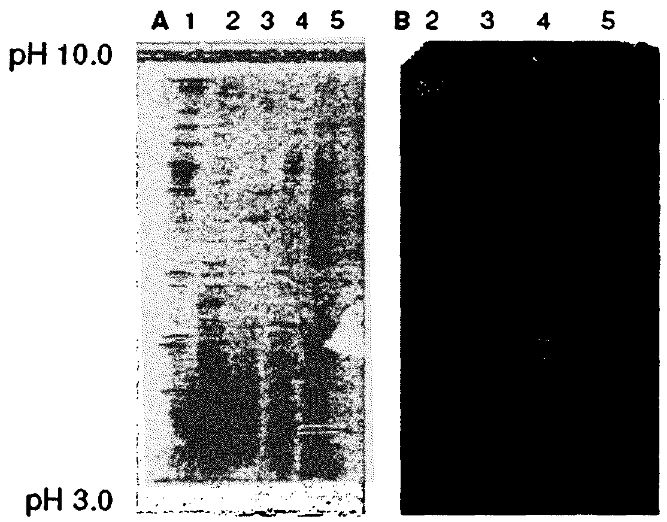


**Figures 6** IEF of secretome (A) and peptidases (B) of *Pleurotus sapidus* (lane 2), *Pleurotus eryngii* (lane 3), *Grifola frondosa* (lane 4), and *Lentinula edodes* (lane 5). Coomassie stained gel (A, pI marker (lane 1)) and activity stained gel (B)

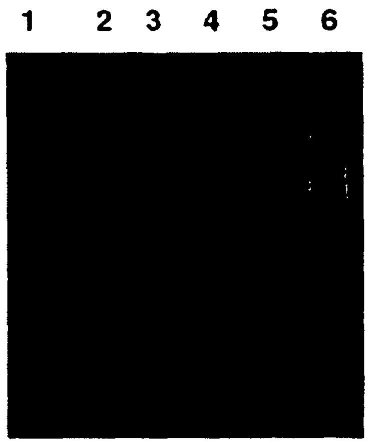


Figure 7 Evaluation of the mechanism of catalysis of the peptidase of *Pleurotus sapidus* using activity stain in the presence of inhibitors. Lane 1: molecular marker; Lane 2: enzyme solution (es); Lane 3: es + 1 mM PMSF; Lane 4: es + 10 mM EDTA; Lane 5: es + 1 mM Pepstatin A; Lane 6: es + 1 mM Aprotinin

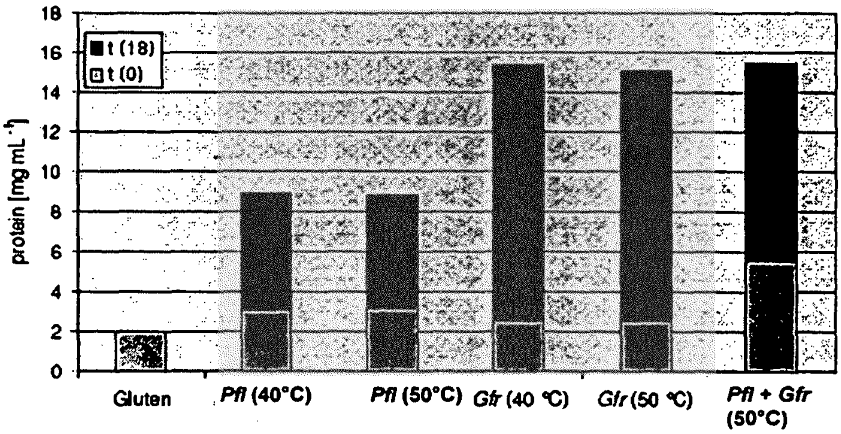


Figure 8 Free protein released by the peptidases of *Pleurotus floridanus* (Pfl) and *Grifola frondosa* (Gfr).

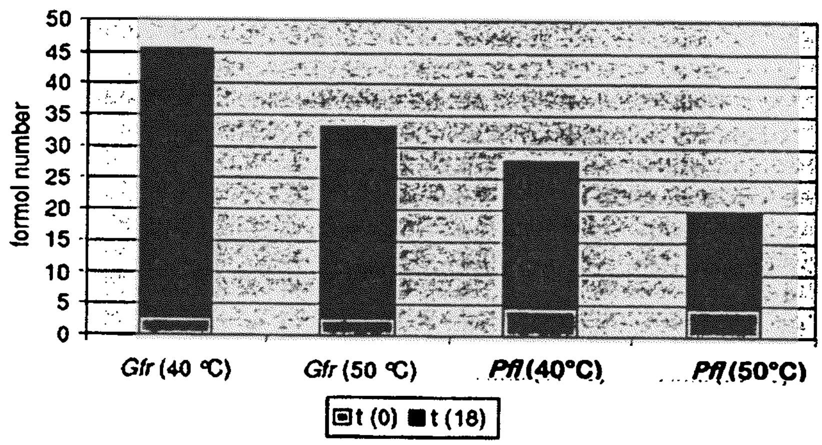


**Figure 9** Formol number of hydrolysates produced using crude peptidases of *Pleurotus floridanus* (Pfl) and *Grifola frondosa* (Gfr).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3694316 A
- EP 1074632 A
- EP 1290951 A
- US 3912822 A
- US 6465209 B
- JP 01291795 A
- WO 2002069733 A
- WO 2004056980 A
- WO 2006104022 A
- JP 2005176620 B
- RU 2003106447
- WO 2002085131 A
- JP 2006075006 A
- JP 2005328738 A
- JP 2006094757 A
- WO 2006103628 A
- WO 2002078461 A
- CN 1397644
- WO 2001076391 A
- WO 2003061675 A
- WO 2002001961 A
- JP 2004229540 A
- EP 07008458 A


### Non-patent literature cited in the description


- **PEDROCHE, J. et al.** *Electronic Journal of Environmental, Agricultural and Food chemistry,* 2003
- **MAEHASHI K. et al.** *Molecular Methods of plant Analysis,* 2002, 47-68
- **LAMSAL, B.P. et al.** *Journal of the American Oil Chemists' Society,* 2006, vol. 83 (8), 731-737
- **JE, J. et al.** *European Food Research and Technology,* 2005, vol. 221 (1-2), 157-162
- **JARUNRATTANASRI, A. et al.** *ACS Symposium Series,* 2005, 83-97
- **SUZUKI, N. et al.** *Phytochemistry,* 2005, vol. 66, 983-990
- **MOREIRA, F.G. et al.** *Brazilian Journal of Microbiology,* 2005, vol. 36 (1), 7-11
- **WANG, H. et al.** *Biochem. Biophys. Res. Commun.,* 2001, vol. 289, 750-755
- **NONAKA, T. et al.** *J. Biol. Chem.,* 1997, vol. 272, 30032-30039
- **HORI, T. et al.** *Acta Crystallogr. D Biol. Chrystallogr.,* 2001, vol. 57, 361-368
- **MINONES CONDE, J. et al.** *Journal of Agricultural and Food Chemistry,* 2005, vol. 53 (20), 8038-8045
- **SCHLICHTHERLE-CERNY, H. et al.** *Journal of Agricultural and Food Chemistry,* 2002, vol. 50 (6), 1515-1522
- **TCHORBANOV, B.** Special Publication. Royal Society of Chemistry, 2001, 491-494
- **ABE, M. et al.** *Biosci. Biotechnol. Biochem.,* 2003, vol. 67, 2018-2021
- **KONG, X. et al.** *Food Chemistry,* 2007, vol. 101, 615-620
- **WANG, J. et al.** *Food Chemistry,* 2007, vol. 101, 1658-1663